# EUROPEAN PATENT APPLICATION

(11) **EP 3 960 170 A1**
(43) Date of publication of application: **02.03.2022**
(21) Application number: 20192551.8
(22) Date of filing: 25.08.2020
(51) Int. Cl.: A61K 31/4196, A61K 45/06, A61P 35/00, A61P 35/02

(54) **DOSING SCHEDULE FOR A METHOD OF TREATMENT WITH DHODH INHIBITORS**

(71) Applicant: Bayer AG, 51373 Leverkusen (DE)
(72) Inventor: The designation of the inventor has not yet been filed
(74) Representative: BIP Patents

(57) **Abstract**

The present invention provides a dosing schedule useful in a method of treatment of hyperproliferative and/or inflammatory disorders, more specifically cancer, with DHODH inhibitors, especially with N-(2-chloro-6-fluorophenyl)-4-[4-ethyl-3-(hydroxymethyl)-5-oxo-4,5-dihydro-1H-1,2,4-triazol-1-yl]-5-fluoro-2-1[(2S)-1,1,1-trifluoropropan-2-yl]oxylbenzamide as well as a method of treatment of hyperproliferative and/or inflammatory disorders, more specifically cancer, with a composition comprising said inhibitors using the new dosing schedule.

## Description

The present invention provides a dosing schedule useful in a method of treatment of hyperproliferative and/or inflammatory disorders, more specifically cancer, with DHODH inhibitors, especially with N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide as well as a method of treatment of hyperproliferative and/or inflammatory disorders, more specifically cancer, with a composition comprising said inhibitors using the new dosing schedule.

### BACKGROUND

Acute myeloid leukemia (AML) is the most common acute leukemia in humans with a 5 year survival of only about 30%. AML is a malignancy of the myeloid line of blood cells. The incidence rates and chances of cure are highly age dependent. The chemotherapy standard of care for AML has not changed significantly over the last decades highlighting the need for novel therapies. A major hallmark of AML is differentiation arrest of the leukemic cells at early stages of cellular differentiation. The potential of leukemic differentiation therapy can be seen with the success of ATRA or arsenic trioxide inducing differentiation in acute promyelocytic leukemia (APL). Around 10% of AML belong to the APL subtype where leukemic cells are harbouring a chromosomal translocation resulting in fusions of oncoproteins involving the retinoic acid receptor. While treatment with ATRA or arsenic trioxide leads to a dramatic increase of patient survival, with overall survival rates of over 70%, unfortunately a comparable differentiation therapy for the non-APL AMLs is lacking (Management of acute promyelocytic leukemia: recommendations from an expert panel on behalf of the European LeukemiaNet, Sanz M.A. et al, Blood 2009, 113(9), 1875-1891). Therefor new therapies inducing differentiation of AML cells are of high interest and medical need.

Dihvdroorotate Dehydrogenase (DHODH) is located in the mitochondria of a cell and is the enzyme responsible for the fourth and rate limiting step in de novo pyrimidine synthesis converting dihydroorotate to orotate (Dihydroorotat-ubiquinone oxidoreductase links mitochondria in the biosynthesis of pyrimidine nucleotides, Löffler, M. et al, Molecular and Cellular Biochemistry 1997, 174, 125-129).

The pyrimidine synthesis is essential for most cancer cells to proliferate and it was recently also linked to differentiation of myeloid malignancies.

Inhibition of DHODH therefore has emerged as a potential treatment for several malignancies such as e.g. breast cancer, SCLC, liver cancer. Currently, inhibition of DHODH with small molecule inhibitors is under clinical investigation for hematological malignancies and lymphomas (NCT03451084; NCT03761069; NCT03760666; NCT03404726; NCT03834584). In the past DHODH inhibitors have also been tested in solid cancer indications using weekly doses or e.g. 5 days on 23 days off schedules for the DHODH inhibitor Brequinar sodium, however were mainly found to be ineffective. Whereas in WO 2017/037022 a dosing of less than or equal to every 72 hours or less than or equal to every 48h for three DHODH inhibitors including Brequinar is disclosed, in WO20123241 a multiphasic dosing regimen is offered for Brequinar for the treatment of cancer but not supported by data and no further clinical trial information is available potentially supporting success of said dosing regimen. This underlines the difficulty to find a dosing schedule for these effectively DHODH inhibiting compounds in order to successfully treat patients suffering from cancer diseases. N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide (BAY 2402234) is a novel highly potent and selective DHODH inhibitor disclosed in (S. Christian et al, Leukemia. 2019;33(10):2403-15, and WO2018/077923). BAY 2402234 has previously been used in daily dosing schedules in preclinical models and initial clinical studies.

### SUMMARY

It has now been found, and this constitutes the basis of the present invention, that the schedules for the administration of the DHODH inhibitor compound N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide (BAY 2402234) show that short off phases of the treatment ensure equal efficacy in preclinical mouse xenograft models, while yielding higher exposure on treatment days. In short, a 4 days on 3 days off schedule as well as a 5 days on 2 days off schedule of BAY 2402234 administration has surprisingly been found to establish comparable efficacy to daily BAY 2402234 dosing, while achieving similar or even higher weekly doses (comparing the total sum of doses given over a 7 day period independent of schedule).

### Definitions

The term "DHODH inhibitor" includes small molecules inhibiting dihydroorotate dehydrogenase, such as e.g. PTC299, Aslan-003 and IMU-838, 6-fluoro-2-(2'-fluoro-[1,1'-biphenyl]-4-yl)-3-methylquinoline-4-carboxylic acid) (brequinar); 5-methyl-N-[4-(trifluoromethyl) phenyl]-isoxazole-4-carboxamide) (leflunomide); (2Z)-2-cyano-3-hydroxy-N-[4-(trifluoromethyl)phenyl]but-2-enamide)(teriflunomide), which can be made according to known methods, as well as those DHODH inhibitors disclosed in WO2018/077923, especially N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, solvates, tautomers, salts, solvates of tautomers, or solvates of salts thereof, particularly N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide a tautomer, an N-oxide, a salt, a salt of a tautomer or a salt of an N-oxide thereof, which can be obtained by methods as disclosed in said WO2018/077923.

The term "daily treatments of equal to 4 days (96hrs) up to 5 days (120 hrs) followed by a short off period of equal to 2 days (48hrs) up to 3 days (72hrs)" includes a treatment for four days or five days and a following off treatment period of two days or three days, as well as treatment for 96hrs to 120hrs followed by a short off period of equal to 48hrs to 72hrs and all combinations thereof. Whenever days are mentioned the shortest unit to count is one day whereas when hours are mentioned the shortest unit is one hour. The schedule can also be expressed as e.g. "four days or five days on/two or three days off" and all combinations thereof.

The term "hyperproliferative disease" includes, but is not limited to, for example : psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include sarcomas, and haematological malignancies including but not limited to leukemias, lymphomas, multiple myeolomas.

Examples of breast cancers include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma and carcinoid tumor.

The term "lung cancer" includes all kinds of carcinoma of the lung, including, but not limited to, small-cell and non-small-cell lung carcinoma, pleuropulmonary blastoma and carcinoid tumor, more especially small cell lung cancer and non-small cell lung cancer.

Examples of brain cancers include, but are not limited to, brain stem and hypophtalmic glioma, glioblastoma, gliosarcoma, glioma, cerebellar and cerebral astrocytoma, medulloblastoma, ependymoma, as well as neuroectodermal and pineal tumour.

The term "reproductive organs" includes male and female reproductive organs. Tumours of the male reproductive organs include, but are not limited to, prostate and testicular cancer. Tumours of the female reproductive organs include, but are not limited to, endometrial, cervical, ovarian, vaginal, and vulvar cancer, as well as sarcoma of the uterus.

Tumours of the digestive tract include, but are not limited to, anal, colon, colorectal, oesophageal, gallbladder, gastric, pancreatic, rectal, small-intestine, and salivary gland cancers.

Tumours of the urinary tract include, but are not limited to, bladder, penile, kidney, renal pelvis, ureter, urethral and human papillary renal cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal, hypopharyngeal, nasopharyngeal, oropharyngeal cancer, lip and oral cavity cancer and squamous cell.

Hematological cancer includes but is not limited to malignancies originating from blood cells, especially white blood cells, more particularly leukemia, lymphoma and meloma.

Leukemias include, but are not limited to acute lymphoblastic leukemia, acute myeloid leukemia, (acute) T-cell leukemia, acute lymphoblastic leukemia, acute lymphocytic leukemia (ALL), acute monocytic leukemia (AML), acute promyelocytic leukemia (APL), bisphenotypic B myelomonocytic leukemia, chronic lymphocytic leukemia, chronic myelogenous leukemia, chronic myeloid leukemia (CML), chronic myelomonocytic leukemia (CMML), large granular lymphocytic leukemia, plasma cell leukemia, and also myelodysplastic syndrome (MDS), which can develop into an acute myeloid leukemia.

Lymphomas include, but are not limited to, AIDS-related lymphoma, chronic lymphocytic lymphoma (CLL), non-Hodgkin's lymphoma (NHL), T-non-Hodgkin lymphoma (T-NHL), subtypes of NHL such as Diffuse Large Cell Lymphoma (DLBCL), activated B-cell DLBCL, germinal center B-cell lymphoma DLBCL, double-hit lymphoma and double-expressor lymphoma; anaplastic large cell lymphoma, B-cell lymphoma, cutaneous T-cell lymphoma, Burkitt's lymphoma, follicular lymphoma, hairy cell lymphoma, Hodgkin's disease, mantle cell lymphoma (MCL), lymphoma of the central nervous system, small lymphocytic lymphoma and chronic lymphocytic lymphoma and Sezary syndrome.

Cancer may be expressed as a solid tumor that may exist in any part or organ of the human body including but not limited to breast, respiratory tract, brain, reproductive organs, digestive tract, urinary tract, eye, liver, skin, head and neck, thyroid, parathyroid and their distant metastases.

The present invention also provides methods of treating angiogenic disorders including diseases associated with excessive and/or abnormal angiogenesis using the new dosing schedule of the present invention.

Inappropriate and ectopic expression of angiogenesis can be deleterious to an organism. A number of pathological conditions are associated with the growth of extraneous blood vessels. These include, for example, diabetic retinopathy, ischemic retinal-vein occlusion, and retinopathy of prematurity, age-related macular degeneration (AMD), neovascular glaucoma, psoriasis, retrolental fibroplasias, angiofibroma, inflammation, rheumatoid arthritis (RA), restenosis, in-stent restenosis, vascular graft restenosis, *etc.* In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer.

Thus, compounds of general formula (I) of the present invention can be utilized to treat and/or prevent any of the aforementioned angiogenesis disorders, for example by inhibiting and/or reducing blood vessel formation; by inhibiting, blocking, reducing, decreasing, *etc.* endothelial cell proliferation, or other types involved in angiogenesis, as well as causing cell death or apoptosis of such cell types by using the new dosing schedule of the present invention.

The term "pharmaceutically acceptable excipient" means inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

### BRIEF DESCRIPTION OF THE DRAWINGS

### Fig1:

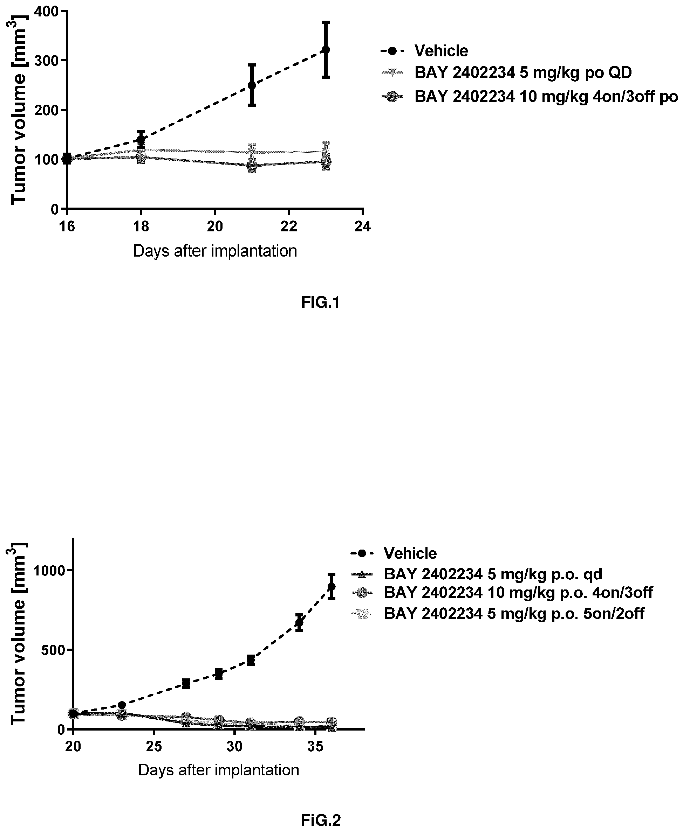

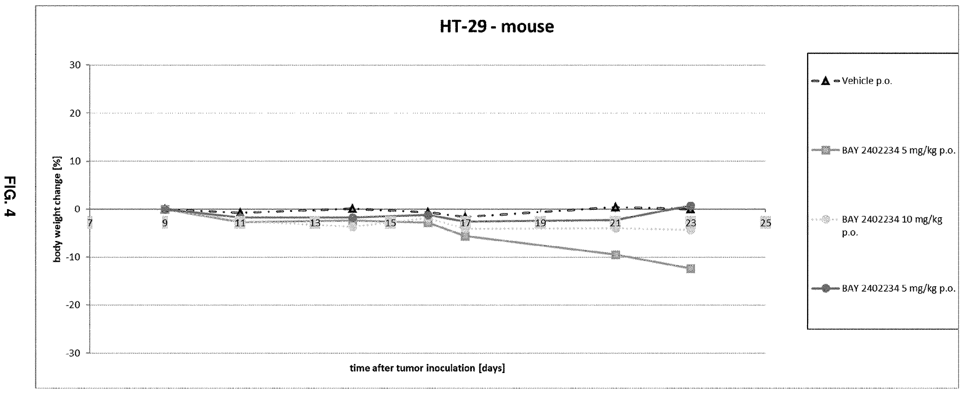

**Figure 1** shows the development of tumor volume over time for the acute myeloid leukemia xenograft model THP1. 16 days post cell inoculation and after randomisation based on tumor size, administration of vehicle compared to daily dosing of 5mg/kg p.o. and 10mg/kg in 4 days on, 3 days off p.o. schedule of BAY 2402234 started. After 7 days of treatment the vehicle control group showed a tripled tumor volume while both BAY 2402234 treatment arms led to a comparable stable disease with basically no further tumor growth after treatment initiation. The weekly dose for the daily schedule sums up to 35 mg/kg, while the weekly dose for the 4 days on, 3 days off schedule sums up to 40 mg/kg.
**Figure 2** shows the development of tumor volume over time for the Mantle cell lymphoma xenograft model Z138. Administration started at day 20 after tumor randomisation comparing vehicle treatments to (1) daily dosing of 5mg/kg p.o. (2) 10mg/kg in 4days on, 3 days off p.o. (3) 5 mg/kg 5 days on, 2 days off p.o. schedule of BAY 2402234. All three BAY 2402234 schedules showed comparable tumor regression in this model while using weekly doses of (1) 35 mg/kg, (2) 40 mg/kg and (3) 25 mg/kg.

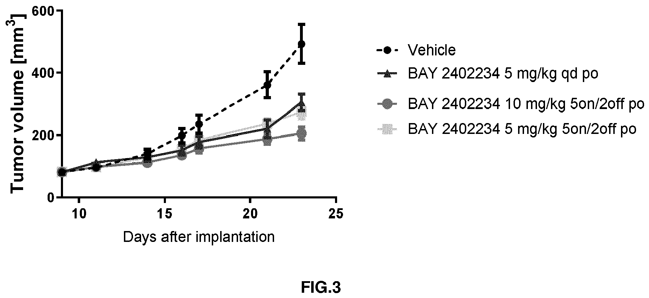

**Figure 3** shows the development of tumor volume over time for the colorectal cancer xenograft model HT29. Administration started at day 9 after tumor randomisation comparing vehicle treatments to (1) daily dosing of 5mg/kg p.o. (2) 10mg/kg in 5 days on, 2 days off p.o. (3) 5 mg/kg 5 days on, 2 days off p.o. schedule of BAY 2402234. All three BAY 2402234 treatments led to tumor growth inhibition, which was comparable for the two 5 mg/kg treatments and slightly stronger in the 10 mg/kg treatment.
   Throughout the experiment the bodyweight was measured and bodyweight behaved comparable throughout the different treatment schedules as shown in Figure 4.
**Figure 4** shows the body weight of vehicle and BAY 2402234 treated animals in the HT29 xenograft model. No significant changes in the body weight. While the vehicle treated animals as well as the 5mg/kg 5 days on 2 days off schedule had a relative constant bodyweight throughout the course of the experiment, the 10 mg/kg 5 days on 2 days off showed around 4% body weight loss at the end of the experiment and the 5 mg/kg daily schedule had a body weight loss at experiments end of above 10%.

### DETAILED DESCRIPTION

The results shown in the experimental section indicate that an intermittent dosing schedule with 2 or 3 off days / week of a DHODH inhibitor can be equally potent to a daily dosing of the same inhibitor with similar or even higher weekly doses. At the same time the intermittent schedule is at least equally tolerated using a similar or even higher weekly dose. Given the higher daily doses in the intermittent schedule and thereby expected higher exposure peaks on treatment days this is a surprising finding. Thereby this should allow to avoid potential adverse events in a clinical setting by allowing patients rest days and a different exposure profile e.g. higher exposure peaks on the "on treatment days" due to a higher dose on a given day compared to the equally stretched daily dosing.

This was somehow unexpected as interruption of dosing is believed to be one major reason the solid cancer trials with DHODH inhibitor Brequinar (which was given weekly or in a 5 days on 23 days off schedule) failed to show sufficient clinical activity.

Thus a first aspect the invention provides a method of treatment of a hyperproliferative disease comprising administering a DHODH inhibitor with a schedule using daily treatments of equal to 96hrs up to 120 hrs followed by a short off period of equal to 48hrs up to 72hrs and a subsequent repetition of the schedule as often as deemed necessary.

In an embodiment the invention provides a method of treatment of a hyperproliferative disease comprising administering a DHODH inhibitor with a schedule using daily treatments of equal to 96hrs followed by a short off period of equal to 48hrs and a subsequent repetition of the schedule as often as deemed necessary.

In an embodiment the invention provides a method of treatment of a hyperproliferative disease comprising administering a DHODH inhibitor with a schedule using daily treatments of equal to 96hrs followed by a short off period of equal to 72hrs and a subsequent repetition of the schedule as often as deemed necessary.

In an embodiment the invention provides a method of treatment of a hyperproliferative disease comprising administering a DHODH inhibitor with a schedule using daily treatments of equal to 120hrs followed by a short off period of equal to 48hrs and a subsequent repetition of the schedule as often as deemed necessary.

In an embodiment the invention provides a method of treatment of a hyperproliferative disease comprising administering a DHODH inhibitor with a schedule using daily treatments of equal to 120hrs followed by a short off period of equal to 72hrs and a subsequent repetition of the schedule as often as deemed necessary.

In an embodiment the invention provides a method of treatment of a hyperproliferative disease comprising administering a DHODH inhibitor with a schedule using daily treatments for 4 days followed by a short off period of 2 days and a subsequent repetition of the schedule as often as deemed necessary.

In an embodiment the invention provides a method of treatment of a hyperproliferative disease comprising administering a DHODH inhibitor with a schedule using daily treatments for 4 days followed by a short off period of 3 days and a subsequent repetition of the schedule as often as deemed necessary.

In an embodiment the invention provides a method of treatment of a hyperproliferative disease comprising administering a DHODH inhibitor with a schedule using daily treatments for 5 days followed by a short off period of 2 days and a subsequent repetition of the schedule as often as deemed necessary.

In an embodiment the invention provides a method of treatment of a hyperproliferative disease comprising administering a DHODH inhibitor with a schedule using daily treatments for 5 days followed by a short off period of 3 days and a subsequent repetition of the schedule as often as deemed necessary.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments of equal to 96hrs up to 120 hrs followed by a short off period of equal to 48hrs up to72hrs and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments of equal to 96hrs followed by a short off period of equal to 48hrs and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments of equal to 96hrs followed by a short off period of equal to 72hrs and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments of equal to 120hrs followed by a short off period of equal to 48hrs and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments of equal to 120hrs followed by a short off period of equal to 72hrs and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments for 4 days followed by a short off period of 2 days and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments for 4 days followed by a short off period of 3 days and a subsequent repetition of the schedule as often as deemed necessary in order to control the disease, achieve its regression or cure the disease mentioned herein.

In some embodiments the invention provides a method of treatment of a hyperproliferative disease comprising administering a DHODH inhibitor with a schedule using daily treatments for 5 days followed by a short off period of 2 days and a subsequent repetition of the schedule as often as deemed necessary in order to control the disease, achieve its regression or cure the disease mentioned herein.

In some embodiments the invention provides a method of treatment of a hyperproliferative disease comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide with a schedule using daily treatments for 5 days followed by a short off period of 3 days and a subsequent repetition of the schedule as often as deemed necessary in order to control the disease, achieve its regression or cure the disease mentioned herein.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 4 to 5 days and
step b) pausing treatment for 2 to 3 days and
step c) optionally repeating steps a) and b) one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 4 to 5 days and
step b) pausing treatment for 2 to 3 days and
step c) optionally repeating steps a) and b) one or more times
   whereby during and/or after treatment the effect of treatment is readily controlled.

In some embodiments the methods of such controlling the effect of treatment are selected from measuring the level of DHO in a blood sample, measuring the count ratio of normal to differentiation arrested cells in a blood sample, and non invasive methods of imaging a solid tumor enabling measuring the seize of the remaining tumor.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 4 to 5 days and
step b) pausing treatment for 2 to 3 days and
step c) optionally repeating steps a) and b) one or more times
   whereby during and/or after treatment the effect of treatment is readily controlled by measurement of DHO in a blood sample.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 4 to 5 days and
step b) pausing treatment for 2 to 3 days and
step c) optionally repeating steps a) and b) one or more times
   whereby during and/or after treatment the effect of treatment is readily controlled and for repeating treatment according to step c) a decision whether or not further treatment is necessary is taken.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts in a method for treatment of cancer following a dosing schedule comprising
step a) administering the compound to a patient daily for 4 days and
step b) pausing treatment for 2 days and
step c) optionally repeating steps a) and b) one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 4 days and
step b) pausing treatment for 3 days and
step c) optionally repeating steps a) and b) one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 5 days and
step b) pausing treatment for 2 days and
step c) optionally repeating steps a) and b) one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvate, salts, solvates of salts in a method for treatment of cancer following a dosing schedule comprising
step a) administering the compound to a patient daily for 5 days and
step b) pausing treatment for 3 days and
step c) optionally repeating steps a) and b) one or more times.

The number of repeated dosing weeks may vary depending on the severity of the disease and the individual condition of the patient.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 to 5 days followed by a recovery period of 2 to 3 days, where the compound is not administered, this schedule being optionally repeated one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 to 5 days followed by a recovery period of 2 to 3 days, where the compound is not administered, this schedule being optionally repeated one or more times as often as deemed necessary in order to control the disease, achieve its regression or cure the disease mentioned herein.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 days followed by a recovery period of 2 days, where the compound is not administered, this schedule being optionally repeated one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 days followed by a recovery period of 2 days, where the compound is not administered, this schedule being optionally repeated one or more times as often as deemed necessary in order to control the disease, achieve its regression or cure the disease mentioned herein.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 days followed by a recovery period of 3 days, where the compound is not administered, this schedule being optionally repeated one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 4 days followed by a recovery period of 3 days, where the compound is not administered, this schedule being optionally repeated one or more times as often as deemed necessary in order to control the disease, achieve its regression or cure the disease mentioned herein.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 5 days followed by a recovery period of 2 days, where the compound is not administered, this schedule being optionally repeated one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 5 days followed by a recovery period of 2 days, where the compound is not administered, this schedule being optionally repeated one or more times as often as deemed necessary in order to control the disease, achieve its regression or cure the disease mentioned herein.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 5 days followed by a recovery period of 3 days, where the compound is not administered, this schedule being optionally repeated one or more times.

In some embodiments the present invention relates to the use of N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, its solvates, salts, solvates of salts for the manufacture of a medicament for the treatment of a hyperproliferative disease, especially a cancer disease, whereby the dosing schedule is daily administration of the compound for 5 days followed by a recovery period of 3 days, where the compound is not administered, this schedule being optionally repeated one or more times as often as deemed necessary in order to control the disease, achieve its regression or cure the disease mentioned herein.

One aspect of the invention is the use of DHODH inhibitors, especially those disclosed in WO WO2018/077923, and more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide for the treatment of cancer applying a 4 days on/3 days off dosing schedule or a 5 days on 2 days off dosing schedule and the respective schedule being optionally repeated one or more times; these compounds for use in the treatment of cancer using a 4 days on/3 days off dosing schedule or a 5 days on/2 days off dosing schedule as well as a method of treatment of cancer diseases comprising administering a specific amount of these compounds while using a 4 days on/3 days off dosing schedule or a 5 days on/2 days off dosing schedule and the respective schedule being optionally repeated one or more times.

One aspect of the invention is the use of DHODH inhibitors, especially those disclosed in WO WO2018/077923, and more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide for the treatment of cancer applying a 4 to 5 days on/2 to 3 days off dosing schedule and the respective schedule being optionally repeated one or more times as often as deemed necessary in order to control the disease, achieve its regression or cure the disease mentioned herein; these compounds for use in the treatment of cancer using a 4 to 5 days on/2 to 3 days off dosing schedule and the respective schedule being optionally repeated one or more times as often as deemed necessary in order to control the disease, achieve its regression or cure the disease mentioned herein, as well as a method of treatment of cancer diseases comprising administering a specific amount of these compounds while using a 4 to 5 days on/2 to 3 days off dosing schedule and the respective schedule being optionally repeated one or more times as often as deemed necessary in order to control the disease, achieve its regression or cure the disease mentioned herein, whereby the specific amount depends on the patient condition and may vary from patient to patient but still within the amount ranges as disclosed herein.

One aspect of the invention is the use of DHODH inhibitors, especially those disclosed in WO WO2018/077923, and more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide for the treatment of cancer applying a 4 days on/3 days off dosing schedule or a 5 days on/2 days off dosing schedule and the respective schedule being optionally repeated one or more times as often as deemed necessary in order to control the disease, achieve its regression or cure the disease mentioned herein; these compounds for use in the treatment of cancer using a 4 days on/3 days off dosing schedule or a 5 days on/2 days off dosing schedule and the respective schedule being optionally repeated one or more times as often as deemed necessary in order to control the disease, achieve its regression or cure the disease mentioned herein, as well as a method of treatment of cancer diseases comprising administering a specific amount of these compounds while using a 4 days on 3 days off dosing schedule or a 5 days on 2 days off dosing schedule and the respective schedule being optionally repeated one or more times as often as deemed necessary in order to control the disease, achieve its regression or cure the disease mentioned herein, whereby the specific amount depends on the patient condition and may vary from patient to patient but still within the amount ranges as disclosed herein.

The DHODH inhibitor may be administered in a pharmaceutical composition, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or a solvate, a tautomer, an N-oxide, a salt, a solvate of a tautomer, a solvate of an N-oxide, a solvate of a salt, particularly a pharmaceutically acceptable salt, or a mixture of same, and one or more excipients, in particular one or more pharmaceutically acceptable excipient(s) a defined herein. Conventional procedures for preparing such pharmaceutical composition in appropriate dosage forms can be utilized.

In some embodiments the DHODH inhibitor may be administered in a pharmaceutical composition, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or solvate, a tautomer, an N-oxide, or a salt, a solvate of a tautomer, a solvate of an N-oxide, a solvate of a salt, thereof, particularly a pharmaceutically acceptable salt, or a mixture of same, and one or more excipients, in particular one or more pharmaceutically acceptable excipient(s) as defined herein for using it in a 4 to 5 days on/2 to 3 days off dosing schedule and the respective schedule being optionally repeated one or more times as often as deemed necessary in order to control the disease, achieve its regression or cure the disease mentioned herein.

In some further embodiments the DHODH inhibitor may be administered in a dosing schedule of 4 days on/3 days off or 5 days on/2 days off dosing schedule in a pharmaceutical composition, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or a solvate, a tautomer, a salt, a solvate of a tautomer, a solvate of a salt, particularly a pharmaceutically acceptable salt, or a mixture of same, and one or more excipients, in particular one or more pharmaceutically acceptable excipient(s) a defined herein for using it in a 4 days on/3 days off dosing schedule or a 5 days on/2 days off dosing schedule and the respective schedule being optionally repeated one or more times as often as deemed necessary in order to control the disease, achieve its regression or cure the disease mentioned herein.

In other embodiments the DHODH inhibitor may be administered in a pharmaceutical composition as mentioned herein, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or a tautomer, thereof, or a mixture of same, and one or more excipients, in particular one or more pharmaceutically acceptable excipient(s) a defined herein for using it in a 4 days on/3 days off dosing schedule or a 5 days on/2 days off dosing schedule and the respective schedule being optionally repeated one or more times as often as deemed necessary in order to control the disease, achieve its regression or cure the disease mentioned herein.

In some embodiments the DHODH inhibitor may be administered in a pharmaceutical composition as mentioned herein, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or a solvate thereof, or a mixture of same, and one or more excipients, in particular one or more pharmaceutically acceptable excipient(s) a defined herein.

In some embodiments the DHODH inhibitor may be administered in a pharmaceutical composition as mentioned herein, in particular comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, or a salt thereof, or a mixture of same, and one or more excipients, in particular one or more pharmaceutically acceptable excipient(s) a defined herein. This composition can be administered in a suitable manner, such as, for example, via the oral, parenteral, pulmonary, nasal, sublingual, lingual, buccal, rectal, vaginal, dermal, transdermal, conjunctival, otic route in a dosing schedule of 4 to 5 days on/ 2 to 3 days off, more especially in a dosing schedule of 4 days on/ 3 days off schedule or in a dosing schedule of 5 days on/ 2 days off.

The present invention furthermore relates to a method of treatment of a hyperproliferative disease, in particular cancer, comprising administering a pharmaceutical composition comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof together with one or more pharmaceutically acceptable excipient(s) in a 4 to 5 days on/ 2 to 3 days off schedule, more especially in a dosing schedule of 4 days on/ 3 days off schedule or in a dosing schedule of 5 days on/ 2 days off.

The present invention furthermore relates to a method of treatment of a hyperproliferative disease, in particular cancer, comprising administering a pharmaceutical composition comprising administering N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof together with one or more pharmaceutically acceptable excipient(s) in a 4 to 5 days on/ 2 to 3 days off schedule, more especially in a 4 days on/ 3 days off schedule or in a 5 days on/ 2 days off schedule.

The present invention furthermore relates to a method of administration of a pharmaceutical composition comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof together with one or more pharmaceutically acceptable excipient(s) in a 4 to 5 days on/ 2 to 3 days off schedule, more especially in a 4 days on/ 3 days off schedule or in a 5 days on/ 2 days off schedule.

The present invention furthermore relates to a method of administration of a DHODH inhibitor, especially N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof together with one or more pharmaceutically acceptable excipient(s) in a 4 to 5 days on/ 2 to 3 days off schedule, more especially in a 4 days on/ 3 days off schedule or in a 5 days on/ 2 days off schedule for treatment of a hyperproliferative disease, especially cancer.

The present invention furthermore relates to a method of administration of a pharmaceutical composition comprising N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof together with one or more pharmaceutically acceptable excipient(s) in a 4 to 5 days on/ 2 to 3 days off schedule, more especially in a 4 days on/ 3 days off schedule or in a 5 days on/ 2 days off schedule for treatment of a hyperproliferative disease, especially cancer.

The present invention furthermore relates to a method of administration of a DHODH inhibitor, especially N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof for use in a treatment schedule of 4 to 5 days on/ 2 to 3 days off.

The present invention furthermore relates to a DHODH inhibitor, especially N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof for use in a treatment schedule of 4 to 5 days on/ 2 to 3 days off.

The present invention furthermore relates to a DHODH inhibitor, especially N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof for use in a treatment schedule of 4 to 5 days on/ 2 to 3 days off for the treatment of a hyperproliferative disease, especially cancer.

In some embodiments the hyperproliferative disease is selected from hyperplasia affecting the skin, psoriasis, keloids; benign prostate hyperplasia (BPH); and a cancer disease of the breast, respiratory tract, brain, reproductive organs, digestive tract, urinary tract, eye, liver, skin, head and neck, thyroid, parathyroid and their distant metastases; sarcomas; haematological malignancies, leukemias, lymphomas, multiple myeolomas.

In some embodiments the hyperproliferative disease is cancer, especially the cancer disease is selected from breast cancer, colorectal carcinoma, gastric cancer, glioblastoma, gliosarcoma, glioma, head & neck cancer, hepatocellular carcinoma, lung cancer, leukemia, acute myeloid leukemia, lymphoma, multiple myeloma, neuroblastoma, ovarian cancer, pancreatic cancer, prostate cancer, renal cell carcinoma, and sarcoma or being any single cancer disease as mentioned herein.

In some embodiments the hyperproliferative disease is cancer, especially the cancer disease is selected from ALL, AML, APL, CMML, DLBCL, MDS, MCL, T-NHL, Burkitt lymphoma, breast cancer, brain cancer, colorectal cancer, glioma, lung cancer, melanoma, ovarian cancer, pancreas cancer, and prostate cancer.

In some embodiments the hyperproliferative disease is cancer, especially the cancer disease is selected from cancer is selected from ALL, AML, APL, CMML, DLBCL, MDS, MCL, T-NHL, colorectal cancer, melanoma and ovarian cancer.

In some embodiments the hyperproliferative disease is cancer, especially the cancer disease is ALL, AML, APL, CMML, DLBCL, MDS, MCL, T-NHL, Burkitt lymphoma, brain cancer, colorectal cancer, ovarian cancer, pancreas cancer, prostate cancer, glioma, non-small cell lung cancer(NSCLC), small cell lung cancer(SCLC), or melanoma.

In some embodiments the hyperproliferative disease is cancer, especially the cancer disease is Acute lymphatic leukemia (ALL), acute myeloid leukemia (AML), acute promyelocytic leukemia (APL), chronic myelomonocytic leukemia (CMML), diffuse large B-cell lymphoma (DLBCL), myeloplastic syndrome (MDS), mantle cell lymphoma (MCL), T-cell non-Hodgkin lymphoma (T-NHL), Burkitt lymphoma, colorectal cancer, lung cancer, melanoma, ovarian cancer and prostate cancer.

In some embodiments the hyperproliferative disease is cancer, especially the cancer disease is acute lymphatic leukemia (ALL), acute myeloid leukemia (AML), acute promyelocytic leukemia (APL), chronic myelomonocytic leukemia (CMML), diffuse large B-cell lymphoma (DLBCL), myeloplastic syndrome (MDS), mantle cell lymphoma (MCL), T-cell non-Hodgkin lymphoma (T-NHL), Burkitt lymphoma.

In some embodiments the hyperproliferative disease is cancer, especially the cancer disease is a hematological cancer, a lymphoma or a solid tumor, especially AML, mantle cell lymphoma or colorectal cancer.

In some embodiments the hyperproliferative disease is cancer, especially the cancer disease is leukemia.

In some embodiments the hyperproliferative disease is cancer, especially the cancer disease is acute lymphatic leukemia (ALL).

In some embodiments the hyperproliferative disease is cancer, especially the cancer disease is acute myeloid leukemia (AML).

In some embodiments the hyperproliferative disease is cancer, especially the cancer disease is acute promyelocytic leukemia (APL).

In some embodiments the hyperproliferative disease is cancer, especially the cancer disease is chronic myelomonocytic leukemia (CMML).

In some embodiments the hyperproliferative disease is cancer, especially the cancer disease is diffuse large B-cell lymphoma (DLBCL).

In some embodiments the hyperproliferative disease is cancer, especially the cancer disease is myeloplastic syndrome (MDS).

In some embodiments the hyperproliferative disease is cancer, especially the cancer disease is mantle cell lymphoma (MCL) T.

In some embodiments the hyperproliferative disease is cancer, especially the cancer disease is lymphoma.

In some embodiments the hyperproliferative disease is cancer, especially the cancer disease is T-cell non-Hodgkin lymphoma (T-NHL).

In some embodiments the hyperproliferative disease is cancer, especially the cancer disease is Burkitt lymphoma.

In some embodiments the hyperproliferative disease is cancer, especially the cancer disease is breast cancer. In some embodiments the hyperproliferative disease is cancer, especially the cancer disease is brain cancer.

In some embodiments the hyperproliferative disease is cancer, especially the cancer disease is colorectal cancer, lung cancer, melanoma, ovarian cancer, and prostate cancer. In some embodiments the hyperproliferative disease is cancer, especially the cancer disease is colorectal cancer. In some embodiments the hyperproliferative disease is cancer, especially the cancer disease is lung cancer, including, but not limited to non-small cell lung cancer and small cell lung cancer. In some embodiments the hyperproliferative disease is cancer, especially the cancer disease is melanoma. In some embodiments the hyperproliferative disease is cancer, especially the cancer disease is ovarian cancer. In some embodiments the hyperproliferative disease is cancer, especially the cancer disease is prostate cancer. The compounds disclosed in WO WO2018/077923, more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, are useful for treatment of diseases or conditions, including chronic diseases, of humans or other species which can be treated or prevented by inhibition of DHODH by a 4 to 3 days on/ 2 to 3 days off dosing schedule, especially a 4 days on 3 days off dosing schedule or a 5 days on 2 days off dosing schedule. These diseases or conditions include (1) inflammatory or allergic diseases such as systemic anaphylaxis and hypersensitivity responses, drug allergies, insect sting allergies and food allergies, (2) inflammatory bowel diseases, such as Crohn's disease, ulcerative colitis, ileitis and enteritis, (3) vaginitis, (4) psoriasis and inflammatory dermatoses such as dermatitis, eczema, atopic dermatitis, allergic contact dermatitis and urticaria, (5) vasculitis, (6) spondyloarthropathies, (7) scleroderma, (8) asthma and respiratory allergic diseases such as allergic asthma, allergic rhinitis, allergic conjunctivitis, hypersensitivity lung diseases and the like, and (9) autoimmune diseases, such as arthritis (including rheumatoid and psoriatic), systemic lupus erythematosus, type I diabetes, glomerulonephritis and the like, (10) graft rejection (including allograft rejection and graft-v-host disease), (11) other diseases in which undesired inflammatory responses are to be inhibited, e.g., atherosclerosis, myositis, neurological disorders such as stroke, ischemic reperfusion injury, traumatic brain injury and closed-head injuries, neurodegenerative diseases (e.g., Parkinson's disease), multiple sclerosis, Alzheimer's disease, encephalitis, meningitis, osteoporosis, gout, hepatitis, nephritis, gall bladder disease, sepsis, sarcoidosis, conjunctivitis, otitis, chronic obstructive pulmonary disease, sinusitis and Behcet's syndrome, and (12) immune diseases or conditions.

In another aspect, the present invention provides methods for treating or preventing viral infections in a subject, the methods comprising administering to a subject having or at risk of developing a viral infection a therapeutically effective amount of a compounds disclosed in WO WO2018/077923, more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide a tautomer, a solvate, a salt, a solvate of a tautomer or a solvate of a salt thereof by using a 4 to 3 days on/ 2 to 3 days off dosing schedule , especially a 4 days on 3 days off dosing schedule or a 5 days on 2 days off dosing schedule the respective schedule being optionally repeated one or more times .

In yet another aspect, the present invention provides methods of treating or preventing Malaria, the method comprising administering to a subject having or at risk of developing Malaria a therapeutically effective amount of a compounds disclosed in WO WO2018/077923, more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide a tautomer, a solvate, a salt, a solvate of a tautomer or a solvate of a salt thereof by using a 4 to 3 days on/ 2 to 3 days off dosing schedule, especially a 4 days on 3 days off dosing schedule or a 5 days on 2 days off dosing schedule the respective schedule being optionally repeated one or more times.

These disorders have been well characterized in humans, but also exist with a similar etiology in other mammals, and can be treated by administering pharmaceutical compositions comprising compounds disclosed in WO WO2018/077923, more specifically N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide a tautomer, a solvate, a salt, a solvate of a tautomer or a solvate of a salt thereof, by using a 4 to 3 days on/ 2 to 3 days off dosing schedule, especially a 4 days on 3 days off dosing schedule or a 5 days on 2 days off dosing schedule the respective schedule being optionally repeated one or more times.

### Experimental Section

### In vivo methods

Tumor cells were injected subcutaneously into the flank of the mice. Treatment was initiated at a predefined tumor size after cell inoculation. BAY 2402234 was applied orally (p.o.) at various dose levels and schedules (daily or intermittent). BAY 2402234 was prepared in solution in PEG400/ETOH 9/1. Animal body weight during the study was monitored at least twice weekly. Subcutaneous tumors were measured with a caliper (length and width) at least twice a week, depending on the doubling time of the tumor. Values are presented as volume. Efficacy was calculated on a specific day according to the formula: T/C (treatment/control ratio) = (mean tumor volume from the treated group/ mean tumor volume from the vehicle group). Tumor volume was calculated using (width² x length)/2.

### Example 1

AML THP1 cells were inoculated in the flank of SCID mice and 16 days after implantation tumors were measured and animals were randomized at an average tumor volume of around 100mm³. Treatment with DHODH Inhibitor BAY 2402234 was started at time of randomization. Either daily dosing of 5 mg/kg or 10 mg/kg at a 4 days on /3 days off schedule was used and the drug was given orally in 90/10 PEG400/EtOH formulation. Both doses are close to the maximal tolerated dose for the used schedule and mouse strain. The daily dosing sums up to a total weekly dose of 35 mg / kg, while the 4 days on/ 3 days off schedule sums up to a 40 mg / kg weekly dose. Efficacy for both doses are comparable in this model and the treatments lead to stable disease (Fig 1).

### Example 2

Mantle Cell Lymphoma Z138 cells were inoculated in the flank of SCID mice and 20 days after implantation tumors were measured and animals were randomized at an average tumor volume of around 100mm³. Treatment with DHODH Inhibitor BAY 2402234 was started at time of randomization. Either daily dosing of 5 mg/kg or 10 mg/kg at a 4 days on /3 days off schedule or 5 mg/kg at a 5 days on 2 days off was used and the drug was given orally in 90/10 PEG400/EtOH formulation. The daily dosing sums up to a total weekly dose of 35 mg / kg, while the 4 days on/ 3 days off schedule sums up to a 40 mg / kg weekly dose and the 5 days on 2 days off sums up to 25 mg/kg weekly dose. Efficacy for all doses are comparable in this model and the treatments lead to shrinkage of the tumors (Fig 2)

### Example 3

Colorectal cancer cells were inoculated in the flank of mice and 9 days after implantation tumors were measured and animals were randomized at an average tumor volume of around 80mm³. Treatment with DHODH Inhibitor BAY 2402234 was started at time of randomization. Either daily dosing of 5 mg/kg or 10 mg/kg at a 5 days on /2 days off schedule or 5 mg/kg at a 5 days on 2 days off was used and the drug was given orally in 90/10 PEG400/EtOH formulation. The daily dosing sums up to a total weekly dose of 35 mg / kg, while the 5 days on/ 2 days off schedule sums up to a 50 or a 25 mg / kg weekly dose respectively. Efficacy for the 5 mg/kg schedules are comparable in this model, while the 10 mg/ kg intermittent schedule is slightly more efficacious. All the treatments lead to tumor growth delay (Fig 3). Looking at the body weight loss as a measure of tolerability the 5 mg/kg 5days on 2 days off was best tolerated followed by 10 mg/kg 5 days on 2 days off and the daily treatment (Fig 4).

## Claims

1. A DHODH inhibitor for use in a method of treatment of a hyperproliferative disease comprising administering a DHODH inhibitor with a dosing schedule using daily treatments of equal to 4 days up to 5 days followed by a short off period of equal to 2 days up to 3 days, or a dosing schedule using daily treatments of equal to 96hrs up to 120 hrs followed by a short off period of equal to 48hrs up to 72hrs to a patient in need thereof and the respective schedule being repeated one or more times.

2. DHODH inhibitor according to claim 1 for use in a treatment schedule of 4 to 5 days on/ 2 to 3 days off.

3. The DHODH inhibitor for use according to claim 1, where the DHODH inhibitor is N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate or a salt, a solvate of a tautomer or a solvate of a salt thereof.

4. The DHODH inhibitor for use according to claim 1 comprising administering the DHODH inhibitor in a pharmaceutical composition comprising in addition one or more pharmaceutically acceptable excipient(s).

5. The DHODH inhibitor for use according claim 4, wherein the DHODH inhibitor is N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide a tautomer, a solvate or a salt, a solvate of a tautomer or a solvate of a salt or a salt of a tautomer.

6. The DHODH inhibitor for use according to claim 1 whereby the dosing schedule is 4 days or 96 hrs on/3 days or 72hrs off.

7. The method according to claim 1 whereby the dosing schedule is 5 days or 120 hrs on, 2 days or 48hrs off.

8. The DHODH inhibitor for use according claim 1, comprising the steps of
step a) administering the DHODH inhibitor to a patient daily for 4 to 5 days and
step b) pausing treatment for 2 to 3 days and
step c) repeating steps a) and b) one or more times.

9. The DHODH inhibitor for use according to claim 8,
whereby during and/or after treatment the effect of treatment is readily controlled by measurement of DHO in a blood sample.

10. The DHODH inhibitor for use according to claim 1, where the hyperproliferative disease is selected from a hyperplasia affecting the skin, psoriasis, keloids; benign prostate hyperplasia (BPH); a cancer disease; sarcomas; and haematological malignancies.

11. The DHODH inhibitor for use according to claims 6 or 7, where the hyperproliferative disease is selected from a hyperplasia affecting the skin, psoriasis, keloids; benign prostate hyperplasia (BPH); and a cancer disease of the breast, respiratory tract, brain, reproductive organs, digestive tract, urinary tract, eye, liver, skin, head and neck, thyroid, parathyroid and their distant metastases; sarcomas; haematological malignancies, leukemias, lymphomas, multiple myeolomas.

12. The DHODH inhibitor for use according to claims 6 or 7, where the hyperproliferative disease is selected from acute lymphatic leukemia, acute myeloid leukemia, acute promyelocytic leukemia, chronic myelomonocytic leukemia, diffuse large B-cell lymphoma, myeloplastic syndrome, mantle cell lymphoma, T-cell non-Hodgkin lymphoma, Burkitt lymphoma, colorectal cancer, melanoma and ovarian cancer

13. The DHODH inhibitor for use according to claims 6 or 7, where the hyperproliferative disease is selected from is ALL, AML, APL, CMML, DLBCL, MDS, MCL, T-NHL, Burkitt lymphoma, brain cancer, colorectal cancer, ovarian cancer, pancreas cancer, prostate cancer, glioma, non-small cell lung cancer(NSCLC), small cell lung cancer(SCLC), or melanoma.

14. A Compound N-(2-chloro-6-fluorophenyl)-4-[4-ethyl-3-(hydroxymethyl)-5-oxo-4,5-dihydro-1H-1,2,4-triazol-1-yl]-5-fluoro-2-{[(2S)-1,1,1-trifluoropropan-2-yl]oxy}benzamide, a tautomer, a solvate, a salt, a solvate of a tautomer or a solvate of a salt thereof for use in a method of administration in a 4 to 5 days on/ 2 to 3 days off schedule for the treatment of a hyperproliferative disease.

15. A compound for use according to claim 14, whereby the hyperproliferative disease is cancer.

16. A compound for use according to claim 14, whereby the hyperproliferative disease is selected from is ALL, AML, APL, CMML, DLBCL, MDS, MCL, T-NHL, Burkitt lymphoma, brain cancer, colorectal cancer, ovarian cancer, pancreas cancer, prostate cancer, glioma, non-small cell lung cancer(NSCLC), small cell lung cancer(SCLC), or melanoma.
